# EUROPÄISCHE PATENTSCHRIFT

(11) **EP 4 316 424 B1**
(45) Veröffentlichungstag und Bekanntmachung des Hinweises auf die Patenterteilung: **05.11.2025**
(21) Anmeldenummer: 23179798.6
(22) Anmeldetag: 16.06.2023
(51) Int. Cl.: A61F 2/18

(54) **VERBESSERTE LÄNGENVERSTELLBARE GEHÖRKNÖCHELCHENPROTHESE MIT IN-SITU-ELONGATION AUS DER KOPFPLATTE HERAUS**
IMPROVED LENGTH-ADJUSTABLE OSSICULAR PROSTHESIS WITH IN SITU ELONGATION OUT OF HEADBOARD
PROTHÈSE D'OSSELETS D'OREILLE À LONGUEUR RÉGLABLE AMÉLIORÉE DOTÉE D'UNE EXTENSION IN SITU DEPUIS LA PLAQUE DE TÊTE

(30) Priorität: 03.08.2022 DE 202022104408 U
(43) Veröffentlichungstag der Anmeldung: 07.02.2024
(73) Patentinhaber: Heinz Kurz GmbH, 72144 Dusslingen (DE)
(72) Erfinder: SANDER, Alexander, 79822 Titisee-Neustadt (DE); STIEGELE, Thomas, 72585 Riederich (DE); MERTENS, Matthias, 24568 Kaltenkirchen (DE); HAAS, Harald, 72116 Mössingen (DE); GÄCKLE, Markus, 75378 Bad Liebenzell (DE)
(74) Vertreter: Kohler Schmid Möbus Patentanwälte

(56) Entgegenhaltungen:
- DE-U1- 29 722 084
- DE-U1- 29 819 892
- US-A1- 2007 255 405
- US-A1- 2012 065 730

## Beschreibung

Die Erfindung betrifft eine Gehörknöchelchenprothese, die mindestens ein Glied oder Teile eines Gliedes der Gehörknöchelchenkette ersetzt oder überbrückt, wobei die Gehörknöchelchenprothese an ihrem einen Ende ein als Kopfplatte zur mechanischen Anlage am Trommelfell und/oder am Hammergriff ausgebildetes erstes Befestigungselement und an ihrem anderen Ende ein zweites Befestigungselement zur mechanischen Verbindung mit einem Glied oder Teilen eines Gliedes der Gehörknöchelchen-Kette oder mit dem Innenohr sowie ein entlang einer Längsachse die beiden Befestigungselemente Schall leitend miteinander verbindendes Verbindungselement umfasst, und wobei das Verbindungselement Stegelemente aufweist, die -zumindest abschnittsweise- radial von der Längsachse weg nach außen mehr oder weniger abgespreizt werden können und dabei die axiale Länge der Gehörknöchelchenprothese mehr oder weniger verkürzen, wobei die Stegelemente einenends direkt in Ankoppelbereiche des ersten Befestigungselements innerhalb der Kopfplattenebene münden und mit diesen beweglich, aber unlösbar verbunden sind, wobei die Stegelemente derart ausgebildet sind, dass sie in einem im menschlichen Mittelohr eingesetzten in-situ-Zustand der Gehörknöchelchenprothese bei Einleitung einer Kraft auf die Stegelemente mit Kraftkomponente parallel zur Längsachse in Richtung vom ersten Befestigungselement zum zweiten Befestigungselement in situ jeweils abschnittsweise eine radial weiter von der Längsachse liegende Position einnehmen und damit die axiale Funktionslänge zwischen dem ersten Befestigungselement und dem zweiten Befestigungselement in situ verkürzen, wobei die Stegelemente bei Einleitung einer Kraft mit Kraftkomponente antiparallel zur Längsachse in Richtung vom zweiten Befestigungselement zum ersten Befestigungselement in situ jeweils abschnittsweise eine radial näher an der Längsachse liegende Position einnehmen und damit die axiale Funktionslänge zwischen dem ersten Befestigungselement und dem zweiten Befestigungselement in situ vergrößern, und wobei die Stegelemente ohne Einwirkung einer Kraft ihre jeweils eingestellte radiale Position zur Längsachse in situ beibehalten.

Eine derartige Vorrichtung ist bekannt aus der DE 297 22 084 U1 (=Referenz [0]).

Eine ähnliche Gehörknöchelchenprothese wird beschrieben in der US 10,687,937 B2 (=Referenz [1]).

### Hintergrund der Erfindung

Gehörknöchelchenprothesen werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell beziehungsweise am Hammergriff anliegt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten GehörknöchelchenProthesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können. (Siehe zum Beispiel DE 42 10 235 C1; EP 0 809 982 B1; US 6,387,128 B1 (=Referenz [2])).

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann.

Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

Um eine solche Flexibilität/Variabilität zu erreichen sind verschiedene Befestigungs- und Ankopplungsvorrichtungen für Gehörknöchelchen, die elastische Teile und/oder Gelenke aufweisen, bekannt. Eine solche gelenkige Verbindung zwischen einem an der Steigbügelfußplatte montierbaren Befestigungselement und dem länglichen Schaft ist beispielsweise in der EP 1 181 907 B1 (=Referenz [3]) beschrieben und wird von der Anmelderin unter dem Markennamen "Ball-Joint" angeboten.

Eine weitere, verschiedentlich auftretende Komplikation entsteht durch eine Unterbelüftung des Mittelohrraumes und damit einhergehende Entzündungen, Tumorbildungen, Verwachsungen im Trommelfellbereich sowie Versteifungen desselben. Beispielsweise bei einer Dysfunktion der Eustachischen Röhre kann es im Mittelohr zu einem Unterdruck kommen, der eine Auskrempung bzw. Ausstülpung (sogenannte Retraktion) des Trommelfells und in der Folge eine Verwachsung etwa mit dem Steigbügel bewirken kann. Um dem entgegen zu wirken und postoperativen Bewegungen des Trommelfells folgen zu können, werden die Kopfplatten bei bekannten Gehörknöchelchenprothesen verkippbar relativ zu dem Verbindungselement gestaltet, das die Kopfplatte mit dem zweiten Befestigungselement verbindet uns meistens als länglicher Schaft ausgeführt ist. Eine solche in sich starre, aber gegenüber dem Verbindungselement verkippbare Kopfplatte ist u.a. beschrieben in der US 2004/0162614 A1 (=Referenz [4]).

Während Referenz [3] eine Gehörknöchelchenprothese, bei welcher die Kopfplatte ein eingebautes, mit dem Verbindungsschaft der beiden Befestigungselemente verbundenes Kugelgelenk aufweist, offenbart EP 1 833 424 B1 (=Referenz [5]) sogar einen als Kugelkette ausgebildeten Verbindungsschaft. Dadurch kann die endgültige axiale Länge der Prothese zumindest abgestuft durch die Auswahl einer bestimmten Anzahl an Kugeln in der Kette und Abschneiden der überzähligen Kugeln festgelegt werden.

Nachteilig bei diesen bekannten Gehörknöchelchenprothesen ist allerdings, dass durch die starre Verkippung der Kopfplatte bei lokalen Medialbewegungen des Trommelfells gleichzeitig die gegenüber liegende Seite der Kopfplatte lateral nach außen bewegt wird, wodurch Druckspitzen auf das Trommelfell erzeugt werden.

Die EP 1 972 307 B1 (=Referenz [6]) offenbart eine Gehörknöchelchenprothese mit hochflexibel gestalteter Kopfplatte, bei welcher einerseits die Vorteile der Prothese gemäß der oben genannten Referenz [5], andererseits aber auch die Vorteile der Referenz [4] beschriebenen Prothesen erhalten bleiben, wobei jedoch die gemeinsamen Nachteile einer starren Verkippung der Kopfplatte vermieden werden. Problematisch bei der Gehörknöchelchenprothese nach Referenz [6] ist allerdings ihre Einführung ins Mittelohr des Patienten, weil insbesondere in radialer Richtung bezüglich ihrer Längsachse die Kopfplatte erheblich seitlich ausragt und nur mittels einer großen künstlichen Öffnung operativ durch den TrommelfellBereich in das Mittelohr eingesetzt werden kann. Diese große Öffnung wächst dann post-operativ natürlich auch schwerer wieder zu und hinterlässt zudem entsprechend große Narben.

Zwar umfasst die Kopfplatte bei Referenz [6] flexible Stegelemente, welche innerhalb der Kopfplatten-Ebene verlaufen und einen radial äußeren Ringbereich der Kopfplatte mit einem radial in der Mitte der Kopfplatte angeordneten zentralen Ankoppelbereich verbinden. Diese Stegelemente sind -zusammen mit dem radial äußeren Ringbereich und dem zentralen Ankoppelbereich- fester Bestandteil der in Referenz [6] offenbarten Kopfplatte. Sie sind geometrisch so gestaltet, dass sie bei lokalen Medialbewegungen des Trommelfells einer solchen Medialbewegung -ebenfalls lokal- folgen, jedoch die Bewegung nicht an entfernte Bereiche der Kopfplatte weitergeben.

Für eine etwaige radiale Stauchung der Kopfplatte innerhalb der Kopfplatten-Ebene und damit einer radialen Verkleinerung des Kopfplatten-Durchmesser, gegebenenfalls zum Zwecke einer erleichterten Durchführung durch das Trommelfell, sind die Stegelemente aber nicht ausgebildet, sondern wären für eine derartige Anwendung ungeeignet. Dies ist insbesondere deswegen so, weil der radial äußere Ringbereich starr ausgeführt ist und daher dem Versuch einer solchen radialen Stauchung nicht folgen oder sich höchstens unkontrollierbar verbiegen würde.

Auch für eine Längenveränderung in Richtung der z-Achse ist die in Referenz [6] beschriebene Gehörknöchelchenprothese absolut ungeeignet, da das Verbindungselement, welches längs der z-Achse verläuft und die Kopfplatte mit dem zweiten Befestigungselement starr verbindet, zwar Schall-übertragend, aber als solches steif ausgebildet ist. Eine Flexibilität der Länge des Verbindungselements in z-Richtung ist bei der Gehörknöchelchenprothese nach Referenz [6] ausgeschlossen und auch gar nicht gewünscht.

Die EP 3 311 773 B1 (=Referenz [7]) beschreibt demgegenüber eine Gehörknöchelchenprothese mit regenschirmartig faltbarer Kopfplatte, mittels welcher die Prothese nahezu minimal-invasiv durch eine kleine Öffnung im Trommelfell operativ ins Mittelohr einsetzbar ist.

Ein weiteres wichtiges Thema bei der Implantation von Gehörknöchelchenprothesen ist die Einstellung der korrekten, an die individuellen Gegebenheiten und Geometrieverhältnisse im Mittelohr des Patienten optimal angepassten axialen Länge der Prothese.

Bereits die oben diskutierte Referenz [5] schlägt dazu eine Kugelkette mit abtrennbaren Endkugeln vor. Dies ermöglicht aber leider keine kontinuierliche, sondern lediglich eine stufenweise Längeneinstellung.

Eine längenvariable Gehörknöchelchenprothese mit einem im Verbindungselement zwischen dem ersten und dem zweiten Befestigungselement eingebauten Verschiebemechanismus zur stufenlosen Längeneinstellung ist in der DE 10 2007 041 539 B4 (=Referenz [8]) beschrieben.

Anstelle einer solchen -in der Herstellung relativ aufwändigen-Verschiebemechanik schlägt die EP 2 238 946 B1 (=Referenz [9]) eine längenvariable Gehörknöchelchenprothese vor, bei welcher im Verbindungselement eine Ziehharmonika-artige Struktur eingebaut ist. Die axiale Länge der Gehörknöchelchenprothese kann dann durch eine axiale Stauchung dieser Struktur verkürzt und durch ein Auseinanderziehen derselben vergrößert werden.

In der EP 2 601 909 B1 (=Referenz [10]) wiederum ist zur axialen Längeneinstellung der Gehörknöchelchenprothese eine Verschiebemechanik im Verbindungselement vorgesehen, welche ein Aufnahmeteil und einen das Aufnahmeteil mit zwei Schenkeln klammerartig umgreifendes Einschiebeteil umfasst, wobei das Aufnahmeteil und das Einschiebeteil relativ zueinander in axialer Richtung des Verbindungselements verfahrbar sind.

Eine Laser-aktivierbare längenvariable Gehörknöchelchenprothese schließlich ist in der EP 3 130 315 B1 (=Referenz [11]) offenbart. Darin wird vorgeschlagen, das Verbindungselement mit streckbaren und/oder stauchbaren, schlaufenartig gefalteten Teilsträngen aus einem Material mit Formgedächtnis aufzubauen. Mit den Schlaufen dieser Teilstränge sind Aktivierungsflächen wärmeleitend verbunden, welche durch Hitzeeinwirkung eine thermische Aktivierung und damit eine Verformung der Schlaufen bewirken können. Auf diese Weise kann die axiale Länge der Gehörknöchelchenprothese verändert und nach Wunsch eingestellt werden.

Die bereits oben zitierte Referenz [1] schließlich offenbart eine Gehörknöchelchenprothese mit einigen der eingangs definierten Merkmalskomplexen. Insbesondere wird vorgeschlagen, im Verbindungselement streifenartige Stegelemente vorzusehen, die radial von der Längsachse weg nach außen abgespreizt werden können und dabei die axiale Länge des Verbindungselements und damit die axiale Länge der Gehörknöchelchenprothese verkürzen. Die Spreizung der Stegelemente erfolgt von der Seite her, also in radialer Richtung bezüglich des Verbindungselements. Eine Vergrößerung der axialen Länge ist bei diesem Wirkmechanismus allerdings nicht vorgesehen. Ohne zusätzliche Maßnahmen ist diese Längeneinstellung auch nur vor dem Einsetzen der Prothese ins Mittelohr des Patienten möglich, nicht jedoch in situ im implantierten Zustand.

### Aufgabe der Erfindung

Aufgabe der vorliegenden Erfindung ist es demgegenüber, eine gattungsgemäße längenverstellbare Gehörknöchelchenprothese der eingangs definierten Art mit möglichst einfachen technischen Mitteln dahingehend zu verbessern, dass unaufwändig und kostengünstig eine Längenverstellung in situ bei bereits platzierter Prothese erfolgen kann, dass die Längenverstellung der Prothese auch von oben her möglich ist, dass dabei eine gleichmäßige und symmetrische Elongation der Prothese sichergestellt wird, und dass eine Anwendung der Erfindung auch etwa bei Partialprothesen mit geringer axialer Bauhöhe und/oder geringer funktionaler Länge oder ermöglicht wird.

### Kurze Beschreibung der Erfindung

Erfindungsgemäß wird diese Aufgabe auf ebenso überraschend einfache wie wirkungsvolle Art und Weise dadurch gelöst, dass das Ankoppelelement Schaft-förmig in Richtung der Längsachse ausgebildet ist, dass sämtliche Stegelemente mit einem Ankoppelelement ebenfalls beweglich und im Betrieb fest, aber lösbar verbunden sind, indem die Stegelemente so ausgebildet sind, dass sie das Ankoppelelement Klammer-artig kraftschlüssig umfassen können, oder indem das Ankoppelelement so ausgebildet ist, dass es kraftschlüssig in die die Stegelemente eingreifen kann, dass das Ankoppelelement seinerseits anderenends entweder mit dem ersten Befestigungselement oder mit dem zweiten Befestigungselement starr verbunden ist, und dass die Stegelemente zusammen mit dem Ankoppelelement das die beiden Befestigungs-elemente Schall leitend miteinander verbindende Verbindungselement bilden.

Derartige Schaft-Bauteile sind besonders einfach herstellbar und mechanisch relativ stabil.

Dadurch können auf relativ simple Weise die Vorteile der oben beschriebenen gattungsgemäßen Gehörknöchelchenprothese, wie sie in Referenz [0] beschrieben ist, genutzt werden, wobei jedoch die Längenänderung bei bereits im Mittelohr platzierter Prothese erfolgen kann, was bei den Prothesen nach den Referenzen [0] oder [1] nicht oder nur mit ganz erheblichen Änderungen möglich wäre. Durch die Möglichkeit zur Umwandlung einer mechanischen Bewegung innerhalb der Fläche der Kopfplatte in eine axiale Längenverstellung, wobei die axiale Bewegung letztlich immer aus der Kopfplattenebene herausführen wird, kann bei der erfindungsgemäßen Gehörknöchelchenprothese eine feinjustierbare Längeneinstellung auch erst nach dem Einsetzen ins Mittelohr, also in situ vorgenommen werden. Die Längenänderung, also die Veränderung des Abstands zwischen der Kopfplatte und dem zweiten Befestigungselement am anderen Ende der Prothese, erfolgt dabei durch eine Expansion aus der Kopfplattenebene heraus in die Tiefe (oder vice versa).

Der erfindungsgemäße Mechanismus ist automatisch innerhalb der Kopfplatte geführt und stellt daher auch ohne zusätzliche Maßnahmen eine gleichmäßige und vor allem symmetrische Elongation sicher. Die Ansätze der im Stand der Technik, insbesondere in Referenz [1] beschriebenen Lösungen erfordern im Gegensatz dazu, dass der Anwender an beiden Seiten die Prothese manipulieren muss um eine symmetrische Längenänderung hervorzurufen.

Da der Mechanismus zur Längenverstellbarkeit in der Kopfplatte integriert und mit dieser verbaut ist, kann die erfindungsgemäße Gehörknöchelchenprothese auch mit einer sehr geringen Bauhöhe hergestellt werden.

Außerdem kann der erfindungsgemäße Mechanismus von oben her in der Kopfplatte bedient werden. Das ist ebenfalls ein wesentlicher Unterschied zum Stand der Technik. So ermöglichen etwa die Prothesen gemäß Referenz [1] nur eine umständliche und relativ viel Raum erfordernde Manipulation von der Seite her. Der erfindungsgemäße Ansatz hingegen umfasst einen äußerst platzsparenden Aufbau und ist auch für endoskopische Zugänge geeignet.

### Bevorzugte Ausführungsformen der Erfindung

Besonders bevorzugte Ausführungsformen der erfindungsgemäßen Gehörknöchelchenprothese zeichnen sich dadurch aus, dass die Stegelemente derart ausgebildet sind, dass ihre jeweils eingestellte radiale Position zur Längsachse in situ durch Einleiten einer entsprechenden Kraft reversibel veränderbar ist.

Insbesondere kann damit die Bewegung der Prothesenteile beim Einstellvorgang reversibel gestaltet und die Prothesenlänge also nicht nur vergrößert, sondern auch wieder verkürzt werden.

Durch den geringen Krafteintrag wird sichergestellt, dass umliegende Mittelohrstrukturen nicht beschädigt werden.

Durch das ausgeklügelte erfindungsgemäße Design der Kopfplatte werden optimale Sichtverhältnisse während des Eingriffs ermöglicht. Durch die Leichtbauweise wird eine optimale Schallübertragung vor allem im Hochtonbereich ermöglicht.

Durch das erfindungsgemäße Kopfplatten Design wurde eine optionale Kopplung an den Hammergriff (Malleus) ermöglicht.

Das erfindungsgemäße Design wurde so entwickelt, dass keine Toträume vorhanden sind. Daher können sich keine Bio-Filme an dem Implantat bilden und Implantat-assoziierte Infektionen ausbilden.

Weitere vorteilhafte Ausführungsformen der Erfindung sind dadurch gekennzeichnet, dass Arretierungseinrichtungen vorhanden sind, welche beim Einleiten einer axialen Kraft in Richtung der Längsachse auf die Stegelemente und/oder auf das Ankoppelelement bei einer oder mehreren axialen Längen des Verbindungselements jeweils einen mechanischen Widerstand hervorrufen.

Die Verstell-Mechanik ist also mit Arretierungspunkten versehen, um eine definierte axiale Länge der Prothese zu erreichen und diese Länge zu fixieren.

Durch den geringen Krafteintrag, wird sichergestellt, dass umliegende Mittelohrstrukturen nicht beschädigt werden.

Durch die Arretierungseinrichtungen, werden optimale Sichtverhältnisse während des Eingriffs ermöglicht.

Eine Wiederaufnahme des Implantats ist während des Eingriffs möglich, um eine Neujustierung zu gewährleisten.

Vorteilhafte Weiterbildungen dieser Ausführungsformen zeichnen sich dadurch aus, dass das Ankoppelelement zumindest in einem im Betrieb den Stegelementen zugewandten Bereich Ring-förmig um den Schaftumfang herum verlaufende, mit axialem Abstand voneinander angeordnete Rillen aufweist, in welche radiale Auskragungen der Stegelemente im implantierten Zustand der Gehörknöchelchenprothese eingreifen können, oder dass die Stegelemente zumindest in einem im Betrieb dem Ankoppelelement zugewandten Bereich mit axialem Abstand voneinander angeordnete Rillen aufweisen, in welche eine radiale Auskragung des Ankoppelelements) im implantierten Zustand der Gehörknöchelchenprothese eingreifen können.

Die Stegelemente sind bei dieser der Rillen-Lösung vorzugsweise konisch dimensioniert, sodass es zu keinem Spiel zwischen den Elementen kommen kann.

Die Rillen können bei bevorzugten Varianten dieser Weiterbildungen definierte, insbesondere identische, axiale Abstände voneinander aufweisen.

Dadurch sind die Ankoppelelemente und Stegelemente besonders leicht herstellbar. Zudem lässt sich damit die endgültige Länge der Gehörknöchelchenprothese präziser einstellen.

Bevorzugt sind auch Ausführungsformen, bei welchen die innerhalb der Kopfplattenebene des ersten Befestigungselements angeordneten Ankoppelbereiche geometrisch so gestaltet sind, dass über sie die Einleitung einer Kraft auf die Stegelemente mit Kraftkomponente parallel oder antiparallel zur Längsachse mittels eines Einstellwerkzeugs erfolgen kann.

Die in-situ Längenverstellung der Gehörknöchelchenprothese lässt sich dadurch erreichen, dass die Struktur innerhalb der Kopfplatte manipuliert wird. Drückt man die Struktur mit einem Instrument zusammen, verlängert sich die Prothese. Spreizt man die Struktur, verkürzt sich die Länge. Das Einstellwerkzeug dient dabei als Implantationshilfe und insbesondere zur Bedienung des Mechanismus zur Längeneinstellung. Es kann als minimalinvasives, insbesondere endoskopisches, Instrument, vorzugsweise Pinzetten-artig oder Zangen-artig, ausgebildet sein.

Eine Längenänderung der Gehörknöchelchenprothese kann prinzipiell durch plastische Verformung der Verbindungsstreben und/oder durch integrierte Gelenke erfolgen.

Eine erste Klasse von Ausführungsformen der erfindungsgemäßen Gehörknöchelchenprothese zeichnet sich dadurch aus, dass die Stegelemente zumindest abschnittsweise, vorzugsweise vollständig mechanisch starr ausgebildet sind.

Damit kann eine gewisse Flexibilität bzw. Variabilität der Prothese erreicht werden, wie sie an sich in der Referenz [3] beschrieben ist. Vorteilhaft im Hinblick auf eine besonders hohe postoperative Beweglichkeit der Prothese sind Weiterbildungen, bei denen eine Vielzahl von aneinander angrenzenden weiteren Drehelementen, vorzugsweise eine Kugelgelenkkette, verwendet werden.

Bei einer zweiten Klasse von dazu alternativen Ausführungsformen der Erfindung sind die Stegelemente zumindest abschnittsweise aus einem plastischen, flexiblen Material ausgebildet, wobei das plastische, flexible Material der Stegelemente insbesondere eine Elastizität ≥ 1%, vorzugsweise eine Elastizität ≥ 2%, aufweist.

Wenn die Stegelemente aus flexiblem Material bestehen, kann der Platz und der technische Aufwand für die mechanischen Drehgelenke sind eingespart werden. Besitzt das Material der Stegelemente eine Flexibilität von >1% kann die Prothese besonders "eng" gebaut werden, wobei auch sichergestellt wird, dass die Prothese kleinsten "topografischen" Änderungen des Trommelfells folgen kann.

Eine erste Gruppe von Weiterbildungen dieser zweiten Klasse von Ausführungsformen, bei welchen das plastische, flexible Material der Stegelemente hochelastisches Material, vorzugsweise amorphes Metall, insbesondere basierend auf Nickel, Eisen, Kobalt oder Zirkonium, und/oder eine Nickel-Titan-Legierung und/oder Memory-Metall, enthält. Die oben genannten Eigenschaften der Flexibilität bei gleichzeitiger für Schallleitung guter Steifigkeit können mit den erwähnten Materialien optimal erreicht werden.

Insbesondere das Material Titan weist neben seiner Festigkeit und ausgezeichneten Schallleitungseigenschaften bekanntermaßen auch eine hervorragende Biokompatibilität am menschlichen Mittelohr auf. Vorteilhaft im Hinblick auf eine postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der EP 1 961 400 B1 (=Referenz [12]) bekannt ist.

Bei einer zweiten Gruppe von Weiterbildungen kann das plastische, flexible Material der Stegelemente aber auch hochelastischen Kunststoff, insbesondere hochfestes elastisches Polymer, und/oder elastische Keramik enthalten.

Diese Werkstoffe ermöglichen, bei hoher Flexibilität und guter Steifigkeit, aber bei geringerer Dichte ideale Voraussetzungen für eine gute Schallübertragung.

Besonders vorteilhaft sind Ausführungsformen, bei welchen die Ankoppelbereiche und die Stegelemente vor dem erstmaligen Einleiten einer Kraft auf die Stegelemente mit Kraftkomponente parallel oder antiparallel zur Längsachse flach innerhalb der Kopfplattenebene des ersten Befestigungselements angeordnet sind.

Damit wird ein besonders kompakter Aufbau der erfindungsgemäßen Gehörknöchelchenprothese ermöglicht.

Bevorzugte Ausführungsformen der Erfindung sind dadurch gekennzeichnet, dass die Stegelemente zwischen ihrem jeweiligen Ankoppelbereich und dem Ankoppelelement kurvenförmig und/oder mäanderförmig und/oder im Zickzack verlaufen.

Durch einen nicht geradlinigen, sondern kurvenförmigen Verlauf verstärkt sich die erwünschte Wirkung einer lokalen Flexibilität der Kopfplatte und ein lediglich lokal begrenztes Ausweichen bei kleineren Medialbewegungen des Trommelfells. Außerdem kann dadurch die Kopfplatte einer eventuellen post-operativen Veränderung des Trommelfells leichter folgen.

Radial äußere, freie Endabschnitte der Stegelemente können bei Ausgestaltungen der Erfindung atraumatische, nicht-spitzzulaufende, bezüglich der Längsachse radial äußere, quer zur Richtung der Längsachse ausgedehnte, freie Endkanten aufweisen oder als atraumatische Flächen, vorzugsweise als geschlossene Flächen oder Ringflächen, insbesondere kreisringförmig oder elliptisch, ausgebildet sein. So können bei einem in der Heilungsphase auftretenden Verkippungsmoment, welches eine erhöhte Kraftwirkung auf die äußeren Endabschnitte zur Folge hat, die Flächenpressung und die Extrusionsgefahr reduziert werden.

Bei weiteren Ausgestaltungen der Erfindung ist das zweite Befestigungselement als Platte, insbesondere als gebogene Platte, als Hülse, als Schlinge, als geschlossene Glocke, insbesondere in Form eines Hohlzylinders, als einfach oder mehrfach geschlitzte Glocke oder als Clip zur mechanischen Verbindung mit einem weiteren Glied der Gehörknöchelchenkette ausgebildet. Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager" dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein zeitlich lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausgestaltung der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen. Die Kopfplatte der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eher eine wachstumshemmende Beschichtung aufweisen.

Alternativ oder ergänzend können bei weiteren Ausführungsformen Teile der erfindungsgemäßen Gehörknöchelchenprothese aus einem Keramikmaterial hergestellt sein. Möglich sind aber auch Ausführungsformen der Erfindung, bei denen die Prothese oder Teile davon aus biokompatiblen Kunststoffen, insbesondere Silikon, Polytetrafluorethylen (PTFE) oder Faserverbund-werkstoffen hergestellt sind. Mit diesen Materialien können postoperative Abstoßungsreaktionen in den meisten Fällen ebenfalls verhindert werden.

In den Rahmen der vorliegenden Erfindung fällt auch ein System umfassend eine erfindungsgemäß aufgebaute Gehörknöchelchenprothese der oben beschriebenen Art sowie ein Einstellwerkzeug zur Manipulation der im Mittelohr eines Patienten eingesetzten Gehörknöchelchenprothese in situ, wobei das Einstellwerkzeug auch als Implantationshilfe dienen kann, und welches als minimalinvasives, insbesondere endoskopisches, Instrument, vorzugsweise Pinzetten-artig oder Zangen-artig, ausgebildet ist.

Vorteilhaft ist hier ein geringeres Trauma bei der operativen Implantation der Gehörknöchelchenprothese, da nur eine kleinere Öffnung des Trommelfells usw. notwendig wird.

Schließlich fällt in den Rahmen der vorliegenden Erfindung auch ein Verfahren zur Implantation einer erfindungsgemäß Gehörknöchelchenprothese der oben beschriebenen Art, welches sich dadurch auszeichnet, dass das erste Befestigungselement, insbesondere die Ankoppelbereiche innerhalb der Kopfplattenebene, Strukturen aus Memory-Metall aufweisen, und dass eine Krafteinleitung auf die Stegelemente mittels, vorzugsweise kontaktloser, insbesondere durch Lichteinstrahlung, beispielsweise Laserstrahlung, hervorgerufener, Erwärmung der Strukturen aus Memory-Metall und deren dadurch erfolgende Verformung bewirkt wird.

Damit wird insbesondere ein berührungsloser Krafteintrag in die erfindungsgemäße Gehörknöchelchenprothese, beispielsweise mittels Wärmestrahlung, ermöglicht.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

### Detaillierte Beschreibung der Erfindung anhand der Zeichnung

In der schematischen Zeichnung sind Ausführungsbeispiele der Erfindung dargestellt, welche in der nachfolgenden Beschreibung näher erläutert werden.

Es zeigen:

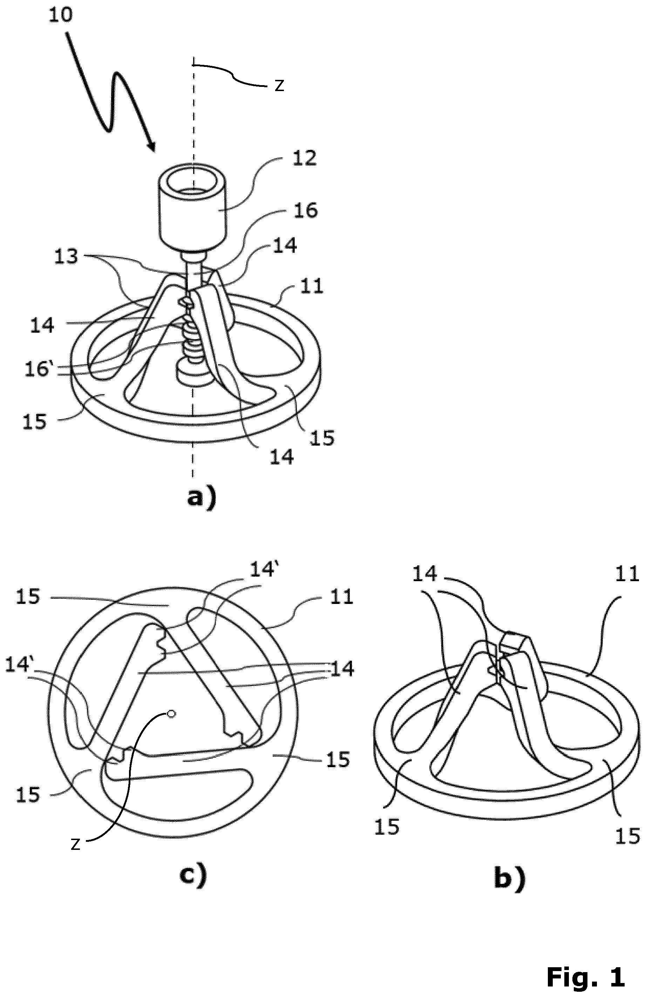

- Fig. 1: drei Abbildungen einer schematischen räumlichen Darstellung einer ersten Ausführungsform der erfindungsgemäßen Gehörknöchelchenprothese, davon zwei Abbildungen in einer Sicht schräg von oben mit Blickrichtung vom zweiten zum ersten Befestigungselement, wobei im Einzelnen dargestellt ist:
a) die komplette Gehörknöchelchenprothese mit einem als einseitig offene, in radialer Richtung geschlossene Glocke gestalteten zweiten Befestigungselement,
b) das als Kopfplatte gestaltete erste Befestigungselement der Gehörknöchelchenprothese von Fig. 1a) mit aus der Kopfplatten-Ebene heraus aufgerichteten Stegen
c) eine Draufsicht in z-Richtung auf die Kopfplatten-Ebene des ersten Befestigungselements von Fig. 1b), wobei jedoch die Stegelemente vor dem Einleiten einer axialen Kraft noch vollständig in der Kopfplatten-Ebene angeordnet sind;

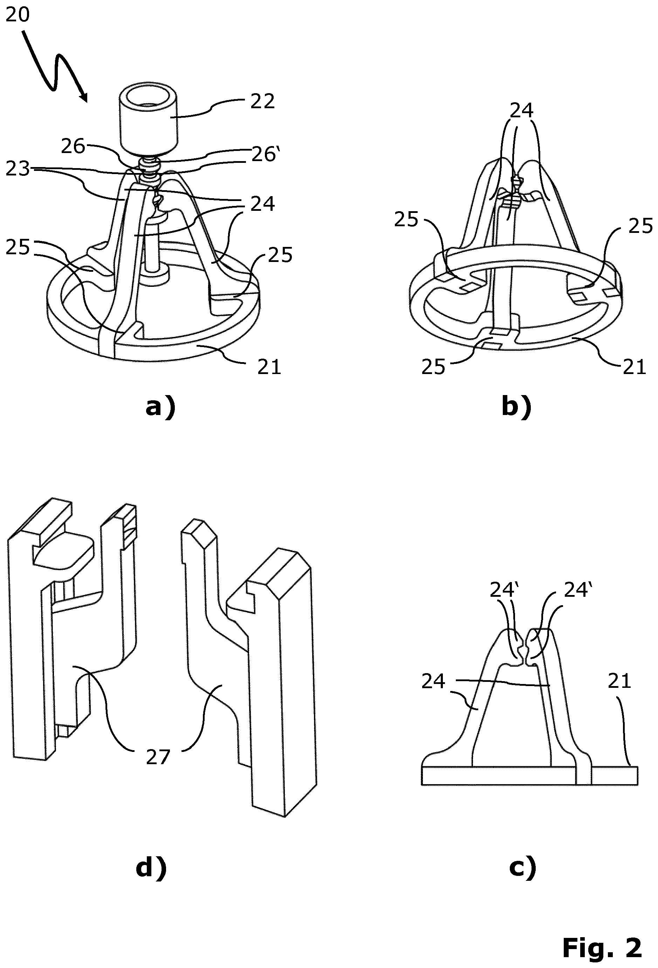

- Fig. 2: eine weitere Ausführungsform einer erfindungsgemäß gestalteten Gehörknöchelchenprothese, nämlich
a) die komplette Gehörknöchelchenprothese in Blickrichtung schräg von oben vom zweiten zum ersten Befestigungselement,
b) das als Kopfplatte gestaltete erste Befestigungselement der Gehörknöchelchenprothese von Fig. 2a) mit aus der Kopfplatten-Ebene heraus aufgerichteten Stegen schräg von unten in Blickrichtung vom ersten Befestigungselement in Richtung der aufgerichteten Stege,
c) die Kopfplatte mit aufgerichteten Stegen von Fig. 2b) mit Blick darauf von der Seite senkrecht zur z-Richtung,
d) ein Einstellwerkzeug zur Manipulation, insbesondere zur Längenverstellung der im Mittelohr eines Patienten eingesetzten Gehörknöchelchenprothese in situ;

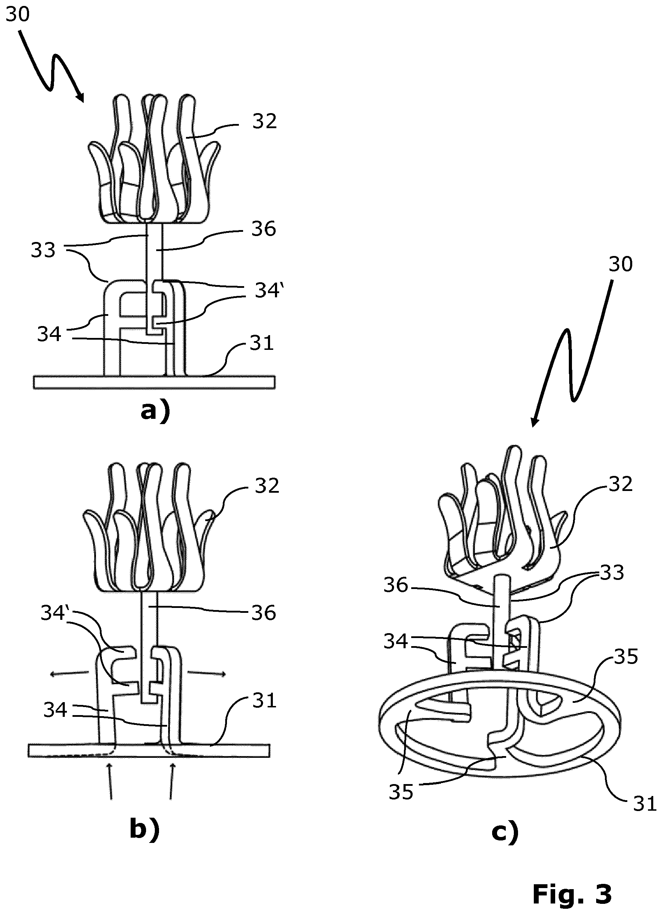

- Fig. 3: eine Ausführungsform einer erfindungsgemäß gestalteten Gehörknöchelchenprothese, bei der das Verbindungselement zwischen dem ersten und zweiten Befestigungselement ein Schaft-förmiges Ankoppelelement ohne Rillen umfasst, wobei das zweite Befestigungselement als geschlitzte Glocke ausgeführt ist, nämlich
a) die Gehörknöchelchenprothese mit seitlich am Ankoppelelement angepressten Stegen in Blickrichtung von der Seite senkrecht zur z-Richtung,
b) die Gehörknöchelchenprothese von Fig. 3a) mit radial vom Ankoppelelement abgehobenen Stegen,
c) die Gehörknöchelchenprothese von Fig. 3b) schräg von unten in Blickrichtung vom ersten Befestigungselement in Richtung zum zweiten Befestigungselement;

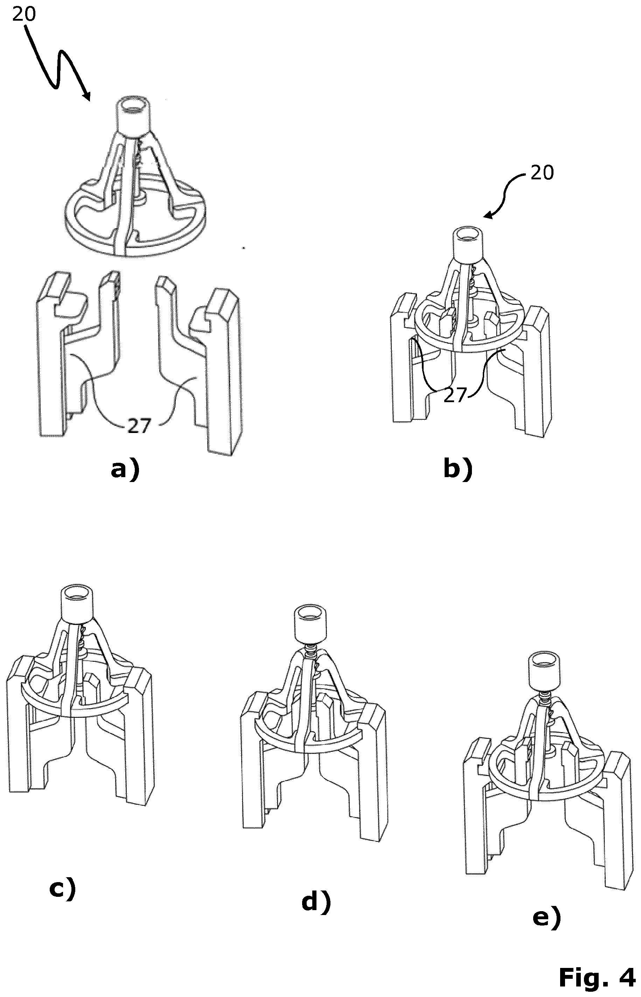

- Fig. 4: die Ausführungsform von Fig. 2a) in sukzessiven Stadien des Einsatzes des Einstellwerkzeugs von Fig. 2d), nämlich
a) mit dem Einsatzwerkzeug in axialem Abstand in z-Richtung von der Gehörknöchelchenprothese,
b) mit auf dem Einsatzwerkzeug aufgesetzter Kopfplatte der Gehörknöchelchenprothese, wobei die Greifarme des Einsatzwerkzeugs noch radial von der Außenkontur der Kopfplatte sowie vom Ankoppelelement beabstandet sind,
c) wie Fig. 4b), jedoch mit radial an der Außenkontur der Kopfplatte sowie am Ankoppelelement angepressten Greifarmen des Einsatzwerkzeugs,
d) wie Fig. 4c), jedoch mit axial vom zweiten Befestigungselement längs der z-Achse in Richtung auf das erste Befestigungselement tiefer am Ankoppelelement angreifenden Stegen,
e) wie Fig. 4d), jedoch mit radial wieder von der Außenkontur der Kopfplatte sowie vom Ankoppelelement gelösten Greifarmen des Einsatzwerkzeugs; und

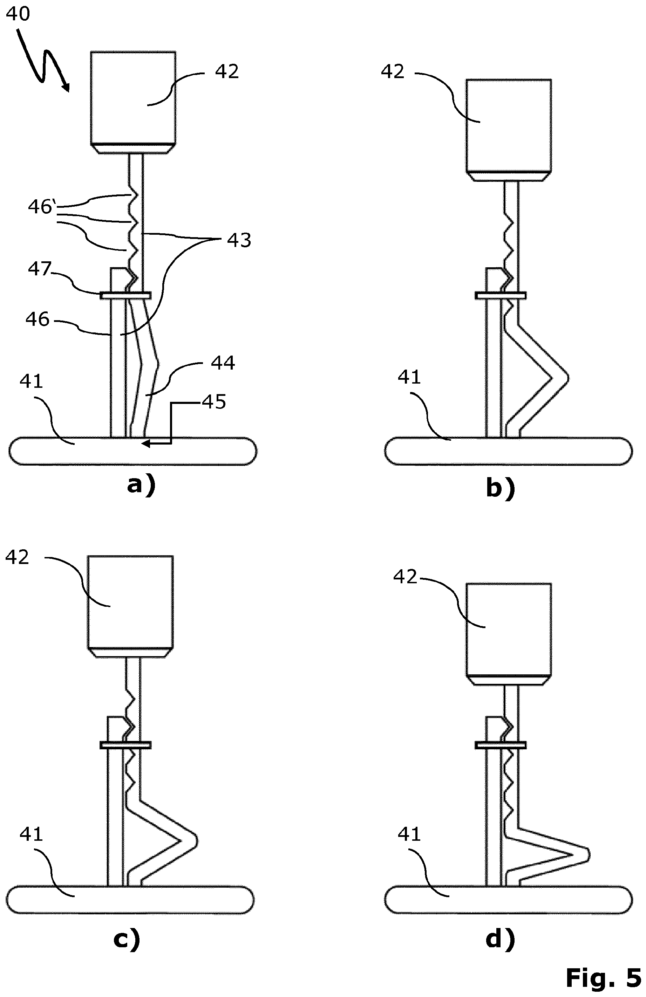

- Fig. 5: vier Abbildungen einer weiteren Ausführungsform mit Blickrichtung von der Seite senkrecht zur z-Achse auf die erfindungsgemäße Gehörknöchelchenprothese, deren Stegelement und Ankoppelelement hier parallel zur z-Richtung angeordnet sind und beide in das erste Befestigungselement münden, wobei das Ankoppelelement radial kraftschlüssig in Rillen des Stegelements eingreift, und zwar in vier unterschiedlichen Relativstellungen von Ankoppelelement und Steg, nämlich
a) mit radialem Eingriff des Ankoppelelements in die dem ersten Befestigungselement nächstliegende Rille des Stegelements,
b) wie Fig. 5a), jedoch mit radialem Eingriff des Ankoppelelements in die dem ersten Befestigungselement zweitnächste Rille des Stegelements,
c) wie Fig. 5b), jedoch mit radialem Eingriff des Ankoppelelements in die dem zweiten Befestigungselement zweitnächste Rille des Stegelements,
d) wie Fig. 5c), jedoch mit radialem Eingriff des Ankoppelelements in die dem zweiten Befestigungselement nächstliegende Rille des Stegelements.

Die in den Figuren der Zeichnung schematisch dargestellten Ausführungsformen der erfindungsgemäßen **Gehörknöchelchenprothese 10; 20; 30; 40** (oder Teilen davon) weisen jeweils am einen Ende ein **erstes Befestigungselement 11; 21; 31; 41** auf, welches in Form einer ebenen Kopfplatte zur mechanischen Anlage am Trommelfell und/oder am Hammergriff ausgebildet ist. Am anderen Ende der Gehörknöchelchenprothese 10; 20; 30; 40 sitzt jeweils ein **zweites Befestigungselement 12; 22; 32; 42** zur mechanischen Verbindung der Prothese mit einem Glied oder Teilen eines Gliedes der Gehörknöchelchen-Kette oder mit dem Innenohr. Dazwischen ist ein jeweils entlang einer **Längsachse z** die beiden Befestigungselemente 11,12 bzw. 21,22 bzw. 31,32 bzw. 41,42 Schall-leitend miteinander verbindendes **Verbindungselement 13; 23; 33; 43** angeordnet.

Das Verbindungselement 13; 23; 33; 43 umfasst jeweils **Stegelemente 14; 24; 34; 44,** die -zumindest abschnittsweise- radial von der Längsachse z weg nach außen mehr oder weniger abgespreizt werden können und dabei die axiale Länge der Gehörknöchelchenprothese 10; 20; 30; 40 mehr oder weniger verkürzen.

Die erfindungsgemäßen Gehörknöchelchenprothesen zeichnen sich dadurch aus, dass die Stegelemente 14; 24; 34; 44 einenends direkt in **Ankoppelbereiche 15; 25; 35; 45** des ersten Befestigungselements 11; 21; 31; 41 innerhalb der Kopfplattenebene münden und mit diesen beweglich, aber unlösbar verbunden sind,
dass sämtliche Stegelemente 14; 24; 34; 44 mit einem **Ankoppelelement 16; 26; 36; 46** ebenfalls beweglich und im Betrieb fest, aber unlösbar verbunden sind, wobei das Ankoppelelement 16; 26; 36; 46 seinerseits anderenends entweder mit dem ersten Befestigungselement 41 oder mit dem zweiten Befestigungselement 12; 22; 32 starr verbunden ist,
dass die Stegelemente 14; 24; 34; 44 zusammen mit dem Ankoppelelement 16; 26; 36; 46 das die beiden Befestigungselemente 11,12; 21,22; 31,32; 41,42 Schall leitend miteinander verbindende Verbindungselement 13; 23; 33; 43 bilden,
dass die Stegelemente 14; 24; 34; 44 derart ausgebildet sind, dass sie in einem im menschlichen Mittelohr eingesetzten in-situ-Zustand der Gehörknöchelchenprothese 10; 20; 30; 40 bei Einleitung einer Kraft auf die Stegelemente 14; 24; 34; 44 mit Kraftkomponente parallel zur Längsachse z in Richtung vom ersten Befestigungselement 11; 21; 31; 41 zum zweiten Befestigungselement 12; 22; 32; 42 in situ jeweils abschnittsweise eine radial weiter von der Längsachse z liegende Position einnehmen und damit die axiale Funktionslänge zwischen dem ersten Befestigungselement 11; 21; 31; 41 und dem zweiten Befestigungselement 12; 22; 32; 42 in situ verkürzen, wobei die Stegelemente 14; 24; 34; 44 bei Einleitung einer Kraft mit Kraftkomponente antiparallel zur Längsachse z in Richtung vom zweiten Befestigungselement 12; 22; 32; 42 zum ersten Befestigungselement 11; 21; 31; 41 in situ jeweils abschnittsweise eine radial näher an der Längsachse z liegende Position einnehmen und damit die axiale Funktionslänge zwischen dem ersten Befestigungselement 11; 21; 31; 41 und dem zweiten Befestigungselement 12; 22; 32; 42 in situ vergrößern,
und dass die Stegelemente 14; 24; 34; 44 ohne Einwirkung einer Kraft ihre jeweils eingestellte radiale Position zur Längsachse z in situ beibehalten.

Die drei **Ansichten a) bis c)** von **Fig. 1** stellen verschiedene Details der Gehörknöchelchenprothese 10 dar:
**Fig. 1a****)** zeigt die komplette Gehörknöchelchenprothese 10 mit einem als axial einseitig offene, in radialer Richtung geschlossene Glocke gestalteten zweiten Befestigungselement 12 sowie einem als Kopfplatte gestalteten ersten Befestigungselement 11, aus welchem jeweils über einen Ankoppelbereich 15 ein Stegelement 14 in z-Richtung auf das zweite Befestigungselement 12 ragt und in radialer Richtung in mechanischem Kontakt zu dem in das zweite Befestigungselement 12 mündenden, Schaft-förmigen Ankoppelelement 16 steht.

In dieser Ausführungsform sind die Stegelemente 14 so ausgebildet sind, dass sie das Ankoppelelement 16 Klammer-artig kraftschlüssig umfassen können. Das Ankoppelelement 16 weist in seinem den Stegelementen 14 zugewandten Bereich ringförmig um den Schaftumfang herum verlaufende, mit axialem Abstand voneinander angeordnete **Rillen 16'** aufweist, in welche **radiale Auskragungen 14'** der Stegelemente 14 eingreifen. Die Auskragungen 14' bilden zusammen mit den Rillen 16' eine Arretierungseinrichtung, welche beim Einleiten einer axialen Kraft in Richtung der Längsachse z auf die Stegelemente 14 und/oder auf das Ankoppelelement 16 bei einer oder mehreren axialen Längen des Verbindungselements 13 jeweils einen mechanischen Widerstand hervorrufen.

**Fig. 1b****)** zeigt lediglich das als Kopfplatte gestaltete erste Befestigungselement 11 der Gehörknöchelchenprothese 10 mit aus der Kopfplatten-Ebene heraus über die Ankoppelbereiche 15 aufgerichteten Stegelementen 14.

**Fig. 1c****)** zeigt eine Draufsicht in z-Richtung auf die Kopfplatten-Ebene des ersten Befestigungselements 11. Hier sind die Stegelemente 14 noch vollständig und flach innerhalb der Kopfplatten-Ebene angeordnet. Die Kopfplatte mit den Ankoppelbereichen 15 und den Stegelementen 14 können als einstückiges Blechbiegeteil ausgeführt sein.

In **Fig. 2** ist eine weitere Ausführungsform einer erfindungsgemäß gestalteten Gehörknöchelchenprothese 20 dargestellt.

**Fig. 2a****)** zeigt wiederum die komplette Gehörknöchelchenprothese 20 mit einem ebenfalls als geschlossene Glocke gestalteten zweiten Befestigungselement 22 sowie einem als Kopfplatte gestalteten ersten Befestigungselement 21, aus welchem jeweils über einen Ankoppelbereich 25 ein Stegelement 24 in z-Richtung auf das zweite Befestigungselement 22 ragt und in radialer Richtung in mechanischem Kontakt zu dem in das zweite Befestigungselement 22 mündenden, Schaft-förmigen Ankoppelelement 26 steht. Auch hier sind wieder die Stegelemente 24 so ausgebildet sind, dass sie das Ankoppelelement 26 Klammer-artig kraftschlüssig umfassen können. Das Ankoppelelement 26 weist wiederum um den Schaftumfang herum verlaufende, mit axialem Abstand voneinander angeordnete **Rillen 26'** auf, in welche **radiale Auskragungen 24'** der Stegelemente 24 eingreifen und zusammen mit den Rillen 26' eine Arretierungseinrichtung bilden.

**Fig. 2b****)** zeigt -diesmal in räumlicher Ansicht schräg von unten in Blickrichtung vom ersten Befestigungselement 21 in Richtung der aufgerichteten Stege 24- wiederum nur das als Kopfplatte gestaltete erste Befestigungselement 21 der Gehörknöchelchenprothese 20 mit aus der Kopfplatten-Ebene heraus über die Ankoppelbereiche 25 aufgerichteten Stegelementen 24.

**Fig. 2c****)** zeigt das erste Befestigungselement 21 von Fig. 2b) mit Blick darauf von der Seite senkrecht zur z-Richtung. Man erkennt an den der Kopfplatte abgewandten Enden der Stegelemente 24 jeweils die radialen Auskragungen 24'.

In **Fig. 2d****)** ist räumlich-schematischer Darstellung ein **Einstellwerkzeug 27** zur in-situ-Längenverstellung der Gehörknöchelchenprothese 20 zu erkennen, dessen Funktionsweise im Detail aus den Abbildungen der Figur 4 verdeutlicht wird.

Die Längenverstellung der Gehörknöchelchenprothese 20 mittels dieses Instruments erfolgt dadurch, dass die Stegelemente 24 radial von der z-Achse weg ausweichen, wenn der Schaft 26 in Achsrichtung verschoben wird. Bei der Montage kann der Schaft seitlich eingeschoben werden. Die Kopfplatte 21 und die Stegelemente 24 sind bei dieser Ausführungsform miteinander verschweißt. Der Schaft mit den Rillen 26' sorgt für eine definierte Längenverstellung und festen Halt.

**Fig. 3** stellt eine weitere Variante einer erfindungsgemäßen Gehörknöchelchenprothese 30 dar. Hier umfasst das Verbindungselement 33 zwischen dem ersten Befestigungselement 31 und dem als geschlitzte Glocke ausgeführten zweiten Befestigungselement 32 ein Schaft-förmiges Ankoppelelement 36 ohne Rillen.

**Fig. 3a****)** zeigt in Blickrichtung von der Seite senkrecht zur z-Richtung die Gehörknöchelchenprothese 30 mit seitlich am Ankoppelelement 36 über **radiale Auskragungen 34'** angepressten Stegelementen 34, während **Fig. 3b****)** die Gehörknöchelchenprothese 30 von Fig. 3a) in einer Betriebsposition mit radial vom Ankoppelelement 36 in Pfeilrichtung abgehobenen Stegelementen 34 darstellt.

**Fig. 3c****)** zeigt die Gehörknöchelchenprothese 30 von Fig. 3b) schräg von unten in Blickrichtung vom ersten Befestigungselement 31 in Richtung zum zweiten Befestigungselement 32. Hier erkennt man gut, wie die Steuerelemente 34 über die Ankoppelbereiche 35 aus der Kopfplatten-Ebene des ersten Befestigungselements 31 zum zweiten Befestigungselement 32 hin herausragen.

Durch eine Klemmverbindung halten die drei um den Umfang verteilt angeordneten Stegelemente 34 das Ankoppelelement 36. Wird durch Druck von unten auf die Kopfplatte 31 diese überwölbt, neigen sich die Stegelemente 34 nach außen und die Klemmverbindung löst sich (temporär). Die Position des Schafts kann dann verändert werden.

Die fünf **Abbildungen a) bis e)** von **Fig. 4** zeigen die Längeneinstellung der Ausführungsform einer erfindungsgemäßen Gehörknöchelchenprothese 20 gemäß Fig. 2a) in sukzessiven Stadien des Einsatzes des Einstellwerkzeugs 27 von Fig. 2d), nämlich
**Fig. 4a****)** die Gehörknöchelchenprothese 20 mit zunächst maximaler axialer Länge in vertikalem Abstand in z-Richtung über dem Einstellwerkzeug 27,
**Fig. 4b****)** die Gehörknöchelchenprothese 20 mit auf dem Einsatzwerkzeug 27 aufgesetzter Kopfplatte, wobei die Greifarme des Einsatzwerkzeugs 27 noch radial von der Außenkontur der Kopfplatte sowie vom Ankoppelelement beabstandet sind,
**Fig. 4c****)** die Gehörknöchelchenprothese 20 wie in Fig. 4b), jedoch mit radial an der Außenkontur der Kopfplatte sowie am Ankoppelelement angepressten Greifarmen des Einsatzwerkzeugs 27,
**Fig. 4d****)** die Gehörknöchelchenprothese 20 wie in Fig. 4c), jedoch mit axial vom zweiten Befestigungselement längs der z-Achse in Richtung auf das erste Befestigungselement tiefer am Ankoppelelement angreifenden Stegen, also mit nunmehr geringerer axialer Länge und
**Fig. 4e****)** die verkürzte Gehörknöchelchenprothese 20 wie in Fig. 4d), jedoch mit radial wieder von der Außenkontur der Kopfplatte sowie vom Ankoppelelement gelösten Greifarmen des Einsatzwerkzeugs 27.

Die beiden Sätze von radial inneren und äußeren Greifarmen des Instruments können aufeinander zubewegt werden. Dadurch wird das Implantat gleichzeitig an der Kopfplatte und am Schaft gegriffen. Jeder Arm ist zweiteilig aufgebaut, wobei je ein Teil am Schaft, ein Teil an der Kopfplatte ansetzt. Die beiden Elemente sind relativ zueinander verschiebbar. Dadurch lässt sich die Länge des Implantats einstellen. Idealerweise bleiben die den Schaft greifenden Elemente relativ in Position zu den Elementen im Mittelohr. Nur die Kopfplatte mit Armen verändert durch die Betätigung des Instruments ihre Position. Somit ändert sich die Länge des Implantats, ohne Druck auf die berührenden Körperteile im Mittelohr (Stapes, etc.) auszuüben.

Die vier **Ansichten a) bis d)** von **Fig. 5** schließlich stellen in vier unterschiedlichen Längeneinstellungen eine weitere Ausführungsform der erfindungsgemäßen Gehörknöchelchenprothese 40, bei welcher das erste Befestigungselement 41 mit dem zweiten Befestigungselement 42 über ein abknickbares Stegelement 44 verbunden ist. Letzteres ist in einem -in der Zeichnung nicht explizit dargestellten- Ankoppelbereich 45 mit dem als ebene Kopfplatte ausgeführten ersten Befestigungselement 41 fest verbunden.

Das Stegelement 44 weist an seinem dem zweiten Befestigungselement 42 zugewandten Ende ringförmig um seinen Umfang verlaufende **Rillen 46'** auf, in die eine radiale **Auskragung 44'** des im Wesentlichen parallel zum Stegelement 44 verlaufenden, im ersten Befestigungselement 41 fest verankerten Ankoppelelements 46 radial kraftschlüssig eingreift. Zusammen bilden das Stegelement 44 und das Ankoppelelement 46 eine Verbindungselement 43.

Ein **Ringelement 47** ist starr am Ankoppelelement 46 befestigt, lässt jedoch durch eine -in der Zeichnung nicht eigens dargestellteringförmige Öffnung das Stegelement 44 in Richtung der z-Achse durchlaufen. Im Betrieb übt das Ringelement 47 also eine radiale Kraft auf das Stegelement 44 aus, sodass letzteres beim axialen Vorbeigleiten am Ankoppelelement 46 diesem nicht radial ausweichen kann, sondern im Betrieb stets an das Ankoppelelement 46 angeschmiegt bleibt.

Durch Einleiten einer Kraft auf das Stegelement 44 in Richtung vom ersten Befestigungselement 41 zum zweiten Befestigungselement 42 kann das Stegelement 44 seitlich, also im Wesentlichen in radialer Richtung von der z-Achse weg abgeknickt werden, wodurch sich der Abstand zwischen erstem Befestigungselement 41 und zweitem Befestigungselement 42 und damit die axiale Länge der Gehörknöchelchenprothese 40 verkürzt. Bei Krafteinleitung antiparallel dazu kann die axiale Länge vergrößert werden.

So kann also durch ein unterschiedlich starkes seitliches Abknicken des Stegelements 44 die axiale Länge der Gehörknöchelchenprothese 40 innerhalb gewisser Grenzen variiert werden. Die Stabilität der Konstruktion wird durch eine Halterung über das im ersten Befestigungselement 41 fest verankerte Ankoppelelement 46 mittels einer Rastverbindung erreicht.

Die vier Abbildungen der Figur 5 zeigen vier unterschiedliche Raststellungen von Ankoppelelement 46 und Stegelement 44, nämlich
**Fig. 5a****)** mit radialem Eingriff des Ankoppelelements 46 in die dem ersten Befestigungselement 41 nächstliegende Rille 46' des Stegelements 44,
**Fig. 5b****)** wie Fig. 5a), jedoch mit radialem Eingriff des Ankoppelelements 46 in die dem ersten Befestigungselement 41 zweitnächste Rille 46' des Stegelements 44,
**Fig. 5c****)** wie Fig. 5b), jedoch mit radialem Eingriff des Ankoppelelements 46 in die dem zweiten Befestigungselement 42 zweitnächste Rille 46' des Stegelements 44, und
**Fig. 5d****)** wie Fig. 5c), jedoch mit radialem Eingriff des Ankoppelelements 46 in die dem zweiten Befestigungselement 42 nächstliegende Rille 46' des Stegelements 44.

### Bezugszeichenliste:

- 10; 20; 30; 40: Gehörknöchelchenprothese
- 11; 21; 31; 41: erstes Befestigungselement
- 12; 22; 32; 42: zweites Befestigungselement
- 13; 23; 33; 43: Verbindungselement
- 14; 24; 34; 44: Stegelemente
- 14'; 24'; 34'; 44': Auskragungen
- 15; 25; 35; 45: Ankoppelbereiche
- 16; 26; 36; 46: Ankoppelelement
- 16'; 26'; 46': Rillen
- 27: Einstellwerkzeug
- 47: Ringelement
- z: Längsachse

### Referenzliste

Für die Beurteilung der Patentfähigkeit in Betracht gezogene Publikationen:
- [0]: DE 297 22 084 U1
- [1]: US 10,687,937 B2
- [2]: DE 42 10 235 C1; EP 0 809 982 B1; US 6,387,128 B1
- [3]: EP 1 181 907 B1
- [4]: US 2004/0162614 A1
- [5]: EP 1 833 424 B1
- [6]: EP 1 972 307 B1
- [7]: EP 3 311 773 B1
- [8]: DE 10 2007 041 539 B4
- [9]: EP 2 238 946 B1
- [10]: EP 2 601 909 B1
- [11]: EP 3 130 315 B1
- [12]: EP 1 961 400 B1

## Patentansprüche

1. Gehörknöchelchenprothese (10; 20; 30; 40), die dazu konfiguriert ist, die mindestens ein Glied oder Teile eines Gliedes der Gehörknöchelchenkette zu ersetzen oder überbrücken, die an ihrem einen Ende ein als ebene Kopfplatte zur mechanischen Anlage am Trommelfell und/oder am Hammergriff ausgebildetes erstes Befestigungselement (11; 21; 31; 41) und an ihrem anderen Ende ein zweites Befestigungselement (12; 22; 32; 42) zur mechanischen Verbindung mit einem Glied oder Teilen eines Gliedes der Gehörknöchelchen-Kette oder mit dem Innenohr sowie ein entlang einer Längsachse (z) die beiden Befestigungselemente (11,12; 21,22; 31,32; 41,42) Schall leitend miteinander verbindendes Verbindungselement (13; 23; 33; 43) umfasst, und wobei das Verbindungselement (13; 23; 33; 43) Stegelemente (14; 24; 34; 44) aufweist, die -zumindest abschnittsweise- radial von der Längsachse (z) weg nach außen abgespreizt werden können und dabei die axiale Länge der Gehörknöchelchenprothese (10; 20; 30; 40) verkürzen, wobei die Stegelemente (14; 24; 34; 44) einenends direkt in Ankoppelbereiche (15; 25; 35; 45) des ersten Befestigungselements (11; 21; 31; 41) innerhalb der Kopfplattenebene münden und mit diesen beweglich, aber unlösbar verbunden sind, wobei die Stegelemente (14; 24; 34; 44) derart ausgebildet sind, dass sie in einem im menschlichen Mittelohr eingesetzten in-situ-Zustand der Gehörknöchelchenprothese (10; 20; 30; 40) bei Einleitung einer Kraft auf die Stegelemente (14; 24; 34; 44) mit Kraftkomponente parallel zur Längsachse (z) in Richtung vom ersten Befestigungselement (11; 21; 31; 41) zum zweiten Befestigungselement (12; 22; 32; 42) in situ jeweils abschnittsweise eine radial weiter von der Längsachse (z) liegende Position einnehmen und damit die axiale Funktionslänge zwischen dem ersten Befestigungselement (11; 21; 31; 41) und dem zweiten Befestigungselement (12; 22; 32; 42) in situ verkürzen, wobei die Stegelemente (14; 24; 34; 44) bei Einleitung einer Kraft mit Kraftkomponente antiparallel zur Längsachse (z) in Richtung vom zweiten Befestigungselement (12; 22; 32; 42) zum ersten Befestigungselement (11; 21; 31; 41) in situ jeweils abschnittsweise eine radial näher an der Längsachse (z) liegende Position einnehmen und damit die axiale Funktionslänge zwischen dem ersten Befestigungselement (11; 21; 31; 41) und dem zweiten Befestigungselement (12; 22; 32; 42) in situ vergrößern, und wobei die Stegelemente (14; 24; 34; 44) ohne Einwirkung einer Kraft ihre jeweils eingestellte radiale Position zur Längsachse (z) in situ beibehalten,
**dadurch gekennzeichnet,**
**dass** ein Ankoppelelement (16; 26; 36; 46) Schaft-förmig in Richtung der Längsachse (z) ausgebildet ist,
**dass** sämtliche Stegelemente (14; 24; 34; 44) mit dem Ankoppelelement (16; 26; 36; 46) beweglich und im Betrieb fest, jedoch lösbar verbunden sind, indem die Stegelemente (14; 24; 34; 44) so ausgebildet sind, dass sie das Ankoppelelement (16; 26; 36) Klammer-artig kraftschlüssig umfassen können,
oder indem das Ankoppelelement (46) so ausgebildet ist, dass es kraftschlüssig in die Stegelemente (44) eingreifen kann,
**dass** das Ankoppelelement (16; 26; 36; 46) seinerseits anderenends entweder mit dem ersten Befestigungselement (41) oder mit dem zweiten Befestigungselement (12; 22; 32) starr verbunden ist,
und **dass** die Stegelemente (14; 24; 34; 44) zusammen mit dem Ankoppelelement (16; 26; 36; 46) das die beiden Befestigungselemente (11,12; 21,22; 31,32; 41,42) Schall leitend miteinander verbindende Verbindungselement (13; 23; 33; 43) bilden.

2. Gehörknöchelchenprothese nach Anspruch 1, **dadurch gekennzeichnet, dass** die Stegelemente (14; 24; 34; 44) derart ausgebildet sind, dass ihre jeweils eingestellte radiale Position zur Längsachse (z) in situ durch Einleiten einer entsprechenden Kraft reversibel veränderbar ist.

3. Gehörknöchelchenprothese nach einem der vorhergehenden Ansprüche, **dadurch gekennzeichnet, dass** Arretierungseinrichtungen vorhanden sind, welche beim Einleiten einer axialen Kraft in Richtung der Längsachse (z) auf die Stegelemente (14; 24; 34; 44) und/oder auf das Ankoppelelement (16; 26; 36; 46) bei einer oder mehreren axialen Längen des Verbindungselements (13; 23; 33; 43) jeweils einen mechanischen Widerstand hervorrufen.

4. Gehörknöchelchenprothese nach Anspruch 3, **dadurch gekennzeichnet, dass** das Ankoppelelement (16; 26; 36) zumindest in einem im Betrieb den Stegelementen (14; 24; 34) zugewandten Bereich Ring-förmig um den Schaftumfang herum verlaufende, mit axialem Abstand voneinander angeordnete Rillen (16'; 26') aufweist, in welche radiale Auskragungen (14'; 24'; 34') der Stegelemente (14; 24; 34) im implantierten Zustand der Gehörknöchelchenprothese (10; 20; 30) eingreifen können, oder dass die Stegelemente (44) zumindest in einem im Betrieb dem Ankoppelelement (46) zugewandten Bereich mit axialem Abstand voneinander angeordnete Rillen (46') aufweisen, in welche eine radiale Auskragung (44') des Ankoppelelements (46) im implantierten Zustand der Gehörknöchelchenprothese (40) eingreifen können.

5. Gehörknöchelchenprothese nach Anspruch 4, **dadurch gekennzeichnet, dass** die Rillen (16; 26'; 46') definierte, vorzugsweise identische, axiale Abstände voneinander aufweisen.

6. Gehörknöchelchenprothese nach einem der vorhergehenden Ansprüche, **dadurch gekennzeichnet, dass** die innerhalb der Kopfplattenebene des ersten Befestigungselements (11; 21; 31; 41) angeordneten Ankoppelbereiche (15; 25; 35; 45) geometrisch so gestaltet sind, dass über sie die Einleitung einer Kraft auf die Stegelemente (14; 24; 34; 44) mit Kraftkomponente parallel oder antiparallel zur Längsachse (z) mittels eines Einstellwerkzeugs (27) in situ erfolgen kann.

7. Gehörknöchelchenprothese nach einem der Ansprüche 1 bis 6, **dadurch gekennzeichnet, dass** die Stegelemente (14; 24; 34; 44) zumindest abschnittsweise, vorzugsweise vollständig mechanisch starr ausgebildet sind.

8. Gehörknöchelchenprothese nach einem der Ansprüche 1 bis 6, **dadurch gekennzeichnet, dass** die Stegelemente (14; 24; 34; 44) zumindest abschnittsweise aus einem plastischen, flexiblen Material ausgebildet sind, und dass das plastische, flexible Material der Stegelemente (14; 24; 34; 44) insbesondere eine Elastizität ≥ 1%, vorzugsweise eine Elastizität ≥ 2%, aufweist.

9. Gehörknöchelchenprothese nach Anspruch 8, **dadurch gekennzeichnet, dass** das plastische, flexible Material der Stegelemente (14; 24; 34; 44) hochelastisches Material, vorzugsweise amorphes Metall, insbesondere basierend auf Nickel, Eisen, Kobalt oder Zirkonium, und/oder eine Nickel-Titan-Legierung und/oder Memory-Metall, enthält.

10. Gehörknöchelchenprothese nach Anspruch 8, **dadurch gekennzeichnet, dass** das plastische, flexible Material der Stegelemente (14; 24; 34; 44) hochelastischen Kunststoff, insbesondere hochfestes elastisches Polymer, und/oder elastische Keramik enthält.

11. Gehörknöchelchenprothese nach einem der vorhergehenden Ansprüche, **dadurch gekennzeichnet, dass** die Ankoppelbereiche (15; 25; 35; 45) und die Stegelemente (14; 24; 34; 44) vor dem erstmaligen Einleiten einer Kraft auf die Stegelemente (14; 24; 34; 44) mit Kraftkomponente parallel oder antiparallel zur Längsachse (z) flach innerhalb der Kopfplattenebene des ersten Befestigungselements (11; 21; 31; 41) angeordnet sind.

12. Gehörknöchelchenprothese nach einem der vorhergehenden Ansprüche, **dadurch gekennzeichnet, dass** die Stegelemente (14; 24; 34; 44) zwischen ihrem jeweiligen Ankoppelbereich (15; 25; 35; 45) und dem Ankoppelelement (16; 26; 36; 46) kurvenförmig und/oder mäanderförmig und/oder im Zickzack verlaufen.

13. System umfassend eine Gehörknöchelchenprothese (10; 20; 30; 40) nach einem der Ansprüche 6 bis 12 sowie ein Einstellwerkzeug (27) zur Manipulation der im Mittelohr eines Patienten eingesetzten Gehörknöchelchenprothese (10; 20; 30; 40) in situ, **dadurch gekennzeichnet, dass** das Einstellwerkzeug (27) als minimalinvasives, insbesondere endoskopisches, Instrument, vorzugsweise Pinzetten-artig oder Zangen-artig, ausgebildet ist.

## Claims

1. An ossicular prosthesis (10; 20; 30; 40) configured to replace or bridge at least one member or parts of a member of the ossicular chain, the ossicular prosthesis (10; 20; 30; 40) comprising, at one end thereof, a first fastening element (11; 21; 31; 41) designed as a flat head plate for mechanical contact with the tympanic membrane and/or with the handle of malleus, and, at its other end, a second fastening element (12; 22; 32; 42) for mechanical connection to a member or parts of a member of the ossicular chain or to the inner ear, and a connecting element (13; 23; 33; 43) which connects the two fastening elements (11,12; 21,22; 31,32; 41,42) to one another in a sound-conducting manner along a longitudinal axis (z), and wherein the connecting element (13; 23; 33; 43) has rib elements (14; 24; 34; 44) which -at least sectionally- can be spread radially outwards away from the longitudinal axis (z), and thereby shorten the axial length of the ossicular prosthesis (10; 20; 30; 40),
wherein the rib elements (14; 24; 34; 44) lead at one end directly into coupling regions (15; 25; 35; 45) of the first fastening element (11; 21; 31; 41) within the head plate plane and are movably, but non-detachably, connected thereto,
wherein the rib elements (14; 24; 34; 44) are designed such that, in an in situ state of the ossicular prosthesis (10; 20; 30; 40) inserted in the human middle ear, with the introduction of a force to the rib elements (14; 24; 34; 44) with the force component in parallel to the longitudinal axis (z) in the direction from the first fastening element (11; 21; 31; 41) to the second fastening element (12; 22; 32; 42), they in each case sectionally assume, in situ, a position located radially further from the longitudinal axis (z) and therefore shorten, in situ, the axial functional length between the first fastening element (11; 21; 31; 41) and the second fastening element (12; 22; 32; 42), the rib elements (14; 24; 34; 44), with the introduction of a force having force components that are anti-parallel with respect to the longitudinal axis (z), in the direction of the second fastening element (12; 22; 32; 42) to the first fastening element (11; 21; 31; 41), in each case sectionally assuming, in situ, a position located radially closer to the longitudinal axis (z), and therefore increasing, in situ, the axial functional length between the first fastening element (11; 21; 31; 41) and the second fastening element (12; 22; 32; 42),
and wherein the rib elements (14; 24; 34; 44) retain, in situ, their adjusted radial position relative to the longitudinal axis (z), when no force acts,
**characterized in**
**that** a coupling element (16; 26; 36; 46) is formed in the shape of a shaft in the direction of the longitudinal axis (z),
**that** all the rib elements (14; 24; 34; 44) are also movably connected to a coupling element (16; 26; 36; 46) and are firmly but detachably connected during operation, whereby the rib elements (14; 24; 34; 44) are designed such that they may encompass the coupling element (16; 26; 36) like a clamp in a force-fit, or whereby the coupling element (46) is designed such that it can engage in the rib elements (44) in a force-fit, that the coupling element (16; 26; 36; 46) in turn is rigidly connected at the other end either to the first fastening element (41) or to the second fastening element (12; 22; 32),
and **that** the rib elements (14; 24; 34; 44) together with the coupling element (16; 26; 36; 46) form the connecting element (13; 23; 33; 43) which connects the two fastening elements (11, 12; 21, 22; 31, 32; 41, 42) to one another in a sound-conducting manner .

2. The ossicular prosthesis according to claim 1, **characterized in that** the rib elements (14; 24; 34; 44) are designed such that their adjusted radial position relative to the longitudinal axis (z) can be reversibly changed in situ by introducing a corresponding force.

3. The ossicular prosthesis according to any of the preceding claims, **characterized in that** locking devices are present which, when an axial force is applied in the direction of the longitudinal axis (z) to the rib elements (14; 24; 34; 44) and/or at the coupling element (16; 26; 36; 46), each cause a mechanical resistance at one or more axial lengths of the connecting element (13; 23; 33; 43).

4. The ossicular prosthesis according to claim 3, **characterized in that** the coupling element (16; 26; 36) has grooves (16'; 26'), which extend around the circumference of the shaft in the form of a ring and are arranged at an axial distance from one another at least in a region facing the rib elements (14; 24; 34) during operation, into which grooves radial projections (14'; 24'; 34') of the rib elements (14; 24; 34) can engage in the implanted state of the ossicular prosthesis (10; 20; 30),
or **in that** the rib elements (44) have grooves (46') arranged at an axial distance from one another at least in a region facing the coupling element (46) during operation, into which grooves a radial projection (44') of the coupling element (46) can engage in the implanted state of the ossicular prosthesis (40).

5. The ossicular prosthesis according to claim 4, **characterized in that** the grooves (16'; 26'; 46') have defined, preferably identical axial distances from one another.

6. The ossicular prosthesis according to any of the preceding claims, **characterized in that** the coupling regions (15; 25; 35; 45) arranged within the head plate plane of the first fastening element (11; 21; 31; 41) are geometrically designed such that they can be used to introduce a force to the rib elements (14; 24; 34; 44) with a force component in parallel or anti-parallel with respect to the longitudinal axis (z) by means of an adjusting tool (27) in situ.

7. The ossicular prosthesis according to any of claims 1 to 6, **characterized in that** the rib elements (14; 24; 34; 44) are designed so as to be mechanically rigid at least sectionally, preferably completely.

8. The ossicular prosthesis according to any of claims 1 to 6, **characterized in that** the rib elements (14; 24; 34; 44) are made of a plastic, flexible material, at least sectionally, and that the plastic, flexible material of the rib elements (14; 24; 34; 44) has, in particular, an elasticity of ≥ 1%, preferably an elasticity of ≥ 2%.

9. The ossicular prosthesis according to claim 8, **characterized in that** the plastic, flexible material of the rib elements (14; 24; 34; 44) contains highly elastic material, preferably amorphous metal, in particular based on nickel, iron, cobalt or zirconium, and/or a nickel-titanium alloy and/or memory metal.

10. The ossicular prosthesis according to claim 8, **characterized in that** the plastic, flexible material of the rib elements (14; 24; 34; 44) contains a highly elastic plastic, in particular a high-strength elastic polymer, and/or elastic ceramic.

11. The ossicular prosthesis according to any of the preceding claims, **characterized in that** the coupling regions (15; 25; 35; 45) and the rib elements (14; 24; 34; 44) are arranged flat within the head plate plane of the first fastening element (11; 21; 31; 41) before the first introduction of a force to the rib elements (14; 24; 34; 44) with a force component in parallel or anti-parallel with respect to the longitudinal axis (z).

12. The ossicular prosthesis according to any of the preceding claims, **characterized in that** the rib elements (14; 24; 34; 44) extend between their respective coupling region (15; 25; 35; 45) and the coupling element (16; 26; 36; 46) in a curved and/or meandering and/or zigzag manner.

13. A system comprising an ossicular prosthesis (10; 20; 30; 40) according to any of claims 6 to 12 and an adjusting tool (27) for manipulating in situ the ossicular prosthesis (10; 20; 30; 40) inserted in the middle ear of a patient, **characterized in that** the adjusting tool (27) is designed as a minimally invasive, in particular endoscopic, instrument, preferably tweezer-like or pincer-like.

## Revendications

1. Prothèse d'osselets auditifs (10; 20; 30; 40) configurée pour remplacer ou ponter au moins un élément ou des parties d'un élément de la chaîne des osselets auditifs, qui comprend à l'une de ses extrémités un premier élément de fixation (11; 21; 31; 41) réalisé sous forme de plaque de tête plane pour l'appui mécanique sur le tympan et/ou sur le manche du marteau et, à son autre extrémité, un second élément de fixation (12; 22; 32; 42) pour la liaison mécanique avec un membre ou des parties d'un membre de la chaîne des osselets ou avec l'oreille interne, ainsi qu'un élément de liaison (13; 23; 33; 43) reliant les deux éléments de fixation (11,12; 21,22; 31,32; 41,42) l'un à l'autre le long d'un axe longitudinal (z) de manière à conduire le bruit,
dans laquelle l'élément de liaison (13; 23; 33; 43) présente des éléments formant entretoises (14; 24; 34; 44) qui peuvent être écartés -au moins dans certaines sections- radialement vers l'extérieur en s'éloignant de l'axe longitudinal (z) et qui permettent ainsi de réduire la longueur axiale de la prothèse d'osselets (10; 20; 30; 40),
dans laquelle les éléments formant entretoises (14; 24; 34; 44) débouchent à une extrémité directement dans des zones d'accouplement (15; 25; 35; 45) du premier élément de fixation (11; 21; 31; 41) à l'intérieur du plan de plaque de tête et sont reliés à celles-ci de manière à être mobiles, mais indissociables, dans laquelle les éléments en formant entretoises (14; 24; 34; 44) sont conçus de telle sorte que, dans un état in situ de la prothèse d'osselets (10; 20; 30; 40) implantée dans l'oreille moyenne humaine, lorsqu'une force est appliquée sur les éléments formant entretoises (14; 24; 34; 44) avec une composante de force parallèle à l'axe longitudinal (z) dans la direction allant du premier élément de fixation (11; 21; 31; 41) vers le deuxième élément de fixation (12; 22; 32; 42) in situ, occupent respectivement par sections une position plus éloignée radialement de l'axe longitudinal (z) et raccourcissent ainsi la longueur fonctionnelle axiale entre le premier élément de fixation (11; 21; 31; 41) et le deuxième élément de fixation (12; 22; 32; 42) in situ,
dans laquelle les éléments formant entretoises (14; 24; 34; 44) prenant, lors de l'application d'une force avec une composante de force antiparallèle à l'axe longitudinal (z), une position plus proche de l'axe longitudinal (z) dans la direction allant du deuxième élément de fixation (12; 22; 32; 42) vers le premier élément de fixation (11; 21; 31; 41) in situ, et les éléments formant entretoises (14; 24; 34; 44) augmentent in situ la longueur fonctionnelle axiale entre le premier élément de fixation (11; 21; 31; 41) et le deuxième élément de fixation (12; 22; 32; 42) in situ, et dans laquelle les éléments formant entretoises (14; 24; 34; 44) conservant in situ leur position radiale respectivement réglée par rapport à l'axe longitudinal (z) sans l'action d'une force,
**caractérisé en ce**
**que** un élément d'accouplement (16; 26; 36; 46) est conçu en forme de tige dans la direction de l'axe longitudinal (z),
**que** tous les éléments formant entretoises (14; 24; 34; 44) sont reliés de manière mobile et, en fonctionnement, de manière fixe mais amovible à l'élément d'accouplement (16; 26; 36; 46), les éléments formant entretoises (14; 24; 34; 44) sont conçus de manière à pouvoir entourer l'élément d'accouplement (16; 26; 36) par adhérence à la manière d'une pince, ou en ce que l'élément d'accouplement (46) est conçu de manière à pouvoir s'engager par adhérence dans les éléments formant entretoises (44), de sorte que l'élément d'accouplement (16; 26; 36; 46) soit relié de manière rigide à l'autre extrémité soit au premier élément de fixation (41) soit au deuxième élément de fixation (12; 22; 32),
et **que** les éléments formant entretoises (14; 24; 34; 44) relient ensemble de manière rigide, avec l'élément d'accouplement (16; 26; 36; 46) pour former l'élément de liaison (13; 23; 33; 43) reliant les deux éléments de fixation (11,12; 21,22; 31,32; 41,42) de manière à conduire le son.

2. Prothèse d'osselets auditifs selon la revendication 1, **caractérisée en ce que** les éléments formant entretoises (14; 24; 34; 44) sont conçus de telle sorte que leur position radiale respective par rapport à l'axe longitudinal (z) peut être modifiée de manière réversible in situ par l'application d'une force correspondante.

3. Prothèse d'osselets auditifs selon l'une des revendications précédentes, **caractérisée en ce qu'**il existe des dispositifs de blocage qui, lors de l'application d'une force axiale dans la direction de l'axe longitudinal (z) sur les éléments formant entretoises (14; 24; 34; 44) et/ou sur l'élément d'accouplement (16; 26; 36; 46) lors d'une ou plusieurs longueurs axiales de l' élément de liaison (13; 23; 33; 43) provoquent respectivement une résistance mécanique.

4. Prothèse des osselets auditifs selon la revendication 3, **caractérisée en ce que** l'élément de couplage (16; 26; 36) présente, au moins dans une zone tournée vers les éléments formant entretoises (14; 24; 34) pendant le fonctionnement, des rainures (16'; 26') qui s'étendent autour de la circonférence de la tige et sont espacées axialement les unes des autres, dans lesquelles des saillies radiales (14'; 24'; 34') des éléments formant entretoises (14; 24; 34) peuvent s'engager à l'état implanté de la prothèse des osselets (10; 20; 30), ou **en ce que** les éléments en forme de entroise (44) présentent, au moins dans une zone tournée vers l'élément de couplage (46) pendant le fonctionnement, des rainures (46') dans lesquelles un saillie radiale (44') de l'élément d'accouplement (46) peut s'engager à l'état implanté de la prothèse des osselets (40).

5. Prothèse d'osselets auditifs selon la revendication 4, **caractérisée en ce que** les rainures (16'; 26'; 46') présentent des distances axiales définies, de préférence identiques, les unes par rapport aux autres.

6. Prothèse des osselets auditifs selon l'une des revendications précédentes, **caractérisée en ce que** les zones de couplage (15; 25; 35; 45) disposées à l'intérieur du plan de la plaque de tête du premier élément de fixation (11; 21; 31; 41) sont conçues géométriquement de telle sorte qu'elles permettent d'exercer in situ une force sur les éléments formant entretoises (14; 24; 34; 44) à l'aide d'un outil de réglage (27), cette force comportant une composante parallèle ou antiparallèle à l'axe longitudinal (z).

7. Prothèse des osselets auditifs selon l'une des revendications 1 à 6, **caractérisée en ce que** les éléments formant entretoises (14; 24; 34; 44) sont réalisés au moins par sections, de préférence entièrement, de manière mécaniquement rigide.

8. Prothèse d'osselets auditifs selon l'une des revendications 1 à 6, **caractérisée en ce que** les éléments formant entretoises (14; 24; 34; 44) sont réalisés au moins par sections dans un matériau plastique flexible, et **en ce que** le matériau plastique flexible des éléments formant entretoises (14; 24; 34; 44) présente en particulier une élasticité ≥ 1 %, de préférence une élasticité ≥ 2 %.

9. Prothèse d'osselets auditifs selon la revendication 8, **caractérisée en ce que** le matériau plastique flexible des éléments formant entretoises (14; 24; 34; 44) contient un matériau hautement élastique, de préférence un métal amorphe, en particulier à base de nickel, de fer, de cobalt ou de zirconium, et/ou un alliage nickeltitane et/ou un métal à mémoire de forme.

10. Prothèse des osselets auditifs selon la revendication 8, **caractérisée en ce que** le matériau plastique souple des éléments formant entretoises (14; 24; 34; 44) contient une matière plastique hautement élastique, en particulier un polymère élastique hautement résistant, et/ou une céramique élastique.

11. Prothèse d'osselets auditifs selon l'une des revendications précédentes, **caractérisée en ce que** les zones de couplage (15; 25; 35; 45) et les éléments formant entretoises (14; 24; 34; 44) sont disposés à plat dans le plan de la plaque de tête du premier élément de fixation (11; 21; 31) avant l'application initiale d'une force sur les éléments formant entretoises (14; 24; 34; 44) avec une composante de force parallèle ou antiparallèle à l'axe longitudinal (z).

12. Prothèse des osselets auditifs selon l'une des revendications précédentes, **caractérisée en ce que** les éléments formant entretoises (14; 24; 34; 44) entre leur zone de couplage respective (15; 25; 35; 45) et l'élément d'accouplement (16; 26; 36; 46).

13. Système comprenant une prothèse d'osselets (10; 20; 30; 40) selon l'une des revendications 6 à 12, ainsi qu'un outil de réglage (27) pour manipuler la prothèse d'osselets (10; 20; 30; 40) implantée dans l'oreille moyenne d'un patient, **caractérisé en ce que** l'outil de réglage (27) est conçu comme un instrument mini-invasif, en particulier endoscopique, de préférence de type pince ou tenaille.
